**Europäisches Patentamt**

**European Patent Office**

**Office européen des brevets**

(11) Veröffentlichungsnummer: **0 156 211 B1**

# (12) EUROPÄISCHE PATENTSCHRIFT

(45) Veröffentlichungstag der Patentschrift: **22.05.91**

(51) Int. Cl.5: **A61M 1/00, A61M 1/10, F04B 43/06**

(21) Anmeldenummer: **85102535.3**

(22) Anmeldetag: **06.03.85**

(54) **Pumpanordnung für medizinische Zwecke.**

(30) Priorität: **07.03.84 DE 3408331**

(43) Veröffentlichungstag der Anmeldung:
**02.10.85 Patentblatt  85/40**

(45) Bekanntmachung des Hinweises auf die
Patenterteilung:
**22.05.91 Patentblatt  91/21**

(84) Benannte Vertragsstaaten:
**DE FR GB IT**

(56) Entgegenhaltungen:
**EP-A- 0 088 900
FR-A- 2 304 357
US-A- 3 449 767**

(73) Patentinhaber: **Fresenius AG
Gluckensteinweg 5
W-6380 Bad Homburg v.d.H.(DE)**

(72) Erfinder: **Polaschegg, Dr.
Grünwiesenweg 9
W-6370 Oberursel 4(DE)**

(74) Vertreter: **Luderschmidt, Wolfgang, Dr.
Dipl.-Chem.
Dr. Fuchs, Dr. Luderschmidt, Dipl.-Phys.
Seids, Dr. Mehler Patentanwälte Abraham-
Lincoln-Strasse 7 Postfach 46 60
W-6200 Wiesbaden(DE)**

Rank Xerox (UK) Business Services

## Beschreibung

Die Erfindung betrifft eine Pumpanordnung für medizinische Zwecke, insbesondere eine Blut- oder Infusionspumpe, mit wenigstens einem Pumpengehäuse, das einen Raum aufweist, der von einer undurchlässigen Membran in einer Arbeitskammer und in eine Antriebskammer unterteilt ist, wobei die Arbeitskammer mit wenigstens einer Öffnung zur Zu- und Abführung des zu fördernden Fluids versehen ist und die Antriebskammer mit Luft als Pumpfluid beaufschlagbar und mit einer ein begrenztes Pumpvolumen aufweisenden Pumpe verbunden ist, und mit einem mit der Antriebskammer verbundenen Ventil für den Druckausgleich bei Hubwechsel der Pumpe.

Eine Pumpanordnung der eingangs erwähnten Art ist aus der DE-A- 32 05 449(& EP-A-0 088 900) bekannt. Sie besteht im wesentlichen aus einem Speichergefäß, das in zwei Kammern unterteilt ist. Die eine Kammer kann dabei mit dem zu pumpenden Fluid, insbesondere Blut, gefüllt und entleert werden, während die andere Kammer alternierend hierzu mit Luft als Pumpfluid beaufschlagt wird. Zwischen beiden Kammern ist eine elastisch erweiterbare, undurchlässige Membran vorgesehen, die gemäß dieser DE-A in Form eines elastischen Schlauchs ausgebildet ist. Beide Kammern sind von einem starren, luftundurchlässigen Pumpengehäuse umgeben, das für die das Pumpfluid aufweisende Kammer eine Öffnung besitzt, die über eine Rohrleitung mit der ein begrenztes Pumpvolumen aufweisenden Pumpe verbunden ist. Diese Pumpe ist als Balgpumpe ausgebildet, die beim Pumpbetrieb zwischen dem oberen und unteren Totpunkt jeweils hin- und herpendelt.

Die mit dem Pumpfluid beaufschlagbare Kammer bzw. der das Pumpfluid aufweisende Raum ist über Unter- und Überdruckventile mit der Umgebung verbunden. Diese Ventile haben die Aufgabe, einen bestimmten Druckwert, nämlich einen bestimmten Überdruck und Unterdruck beim Betrieb der Pumpe einzuhalten. Wird beispielsweise die Pumpe blockiert oder treten die üblichen Anlaufschwierigkeiten auf, so erfolgt lediglich ein Zu- oder Abpumpen des Pumpfluids bis zu dem durch die Ventile gesteuerten Druckwert, die üblicherweise selbsttätig arbeiten.

In der Regel erfolgt der Pumpenbetrieb jedoch so, daß die Über- und Unterdruckventile nicht betätigt werden. Bei einer solchen Arbeitsweise wird also das Pumpfluid bei der Entleerung der Pumpenkammer zusammengepreßt (Überdruck) und bei der Füllung der Pumpenkammer expandiert (Unterdruck). Hier stellen sich jedoch Probleme beim Betrieb der Pumpanordnung ein, die sich vor allen Dingen auf die Pumpkapazität dieser Anordnung auswirken.

Nachstehend wird die Wirkungsweise der bekannten Pumpe und deren Problematik erläutert.

Wie bereits vorstehend festgestellt, wird der Druck in der Pumpe zwischen einem unteren Druckwert $p_{min}$ und einem oberen Druckwert $p_{max}$ schwanken, wobei gerade noch nicht die Ventile sich öffnen werden.

Nun wird abhängig vom Einsatz der Pumpe ein bestimmter Ansaugdruck $p_E$ bzw. Förderdruck $p_K$ benötigt, die an die Pumpanordnung anzulegen sind.

Setzt man nun voraus, daß man für die Membran keine Kraft zur Expansion benötigt, dann gilt für den Grenzfall $p_E = p_{min}$ bzw. $p_K = p_{max}$.

Das Pumpverhalten der bekannten Pumpe wird nun an folgendem Beispiel erläutert: $p_E = -400$ mbar und $p_K = +600$ mbar, während das Innenvolumen der Pumpenkammer $V_1 = 50$ cm$^3$ und das Balgvolumen der Balgpumpe $V_2 = 30$ cm$^3$ betragen sollen.

Im Ausgangszustand ist $V_1$ (komprimiert) = 0 cm$^3$ und $V_2 = 30$ cm$^3$, während $p = p_{max} = +600$ mbar absolut = 1.600 mbar ist.

Anschließend wird angesaugt, wobei $V_1$ gegen 50 cm$^3$ und $V_2$ gegen 0 cm$^3$ geht. Somit wird das Volumen von 30 auf 50 cm$^3$ expandiert.

Über die Beziehung P x V = constant folgt:
$1.600 \times 30 = p_E \times 50$
$p_E = 960$ mbar

Demzufolge beträgt also der Expansionsunterdruck $P_E$ lediglich -40 mbar abs., was zum Betrieb der Pumpe nicht zufriedenstellend ist.

Sollen nunmehr bei der Expansion der Balgpumpe -400 mbar erreicht werden, so wird lediglich - wie nachstehend erläutert - ein geringeres Pumpvolumen erzielt. Denn über die vorstehende Beziehung ergibt sich:
$1.600 \times 30 = 600 \times V_T$ ($V_T$ = erforderliches Balgvolumen)
$V_T = 80$ cm$^3$
d.h. im Grenzfall kann zwar gerade der Ansaugunterdruck erreicht werden, wobei jedoch allerdings das Balgvolumen von 50 cm$^3$ für ein vollständiges Abpumpen nicht ausreicht. Hierzu würden 80 cm$^3$ benötigt.

Eine weitere Pumpanordnung ist aus der US-A-35 68 214 bekannt, bei der zwei Pumpkammern wechselseitig von einer Pumpe mit Pumpfluid beaufschlagt werden, wobei als Pumpfluid isotomische Kochsalzlösung und Quecksilber eingesetzt werden. Die bei solchen Systemen auftretende Verschiebung der Phasenlage bei Hubwechsel der Pumpe, beeinflußt die Wirkung der Pumpe erheblich und läßt sich im allgemeinen nicht in den Griff bekommen, so daß sich diese Pumpe in der Praxis als nicht einsetzbar erwiesen hat.

Der Erfindung liegt daher die Aufgabe zugrunde, eine Pumpanordnung der eingangs erwähnten Art zur Verfügung zu stellen, die innerhalb der eingestellten Druckgrenzen die Pumpenkammer im Betrieb vollständig füllt und entleert.

Die Lösung der Aufgabe erfolgt dadurch, daß ein Steuergerät vorgesehen ist, das vor jedem Hubwechsel der Pumpe das Ventil öffnet und nach Druckausgleich auf Umgebungsdruck schließt und danach den nächsten Hub der Pumpe einleitet.

Die erfindungsgemäße Pumpanordnung weist gegenüber der bekannten Pumpanordnung den Vorteil auf, daß bei gegebenen Volumina des Pumpengehäuses und der Pumpe (also dem Innenvolumen des Pumpgehäuses und des Balgvolumens) die vorgegebenen Druckgrenzen zwangsläufig erreicht werden, also die Pumpe völlig zufriedenstellend arbeitet.

Dies ist darauf zurückzuführen, daß der jeweilige Ausgangsdruck der Atmosphärendruck, also 1000 mbar, ist.

Demzufolge wird bei den eingangs erwähnten Maßnahmen ($V_1$ = 50, $V_2$ = 30, $p_{min}$ = -400 mbar und $p_{max}$ = 600 mbar) folgendes Pumpergebnis erzielt:

a) Pumpe am unteren Totpunkt

$V_1$ = 0 cm$^3$

$V_2$ = 30 cm$^3$

p = p abs. = 1000 mbar.

Beim Expandieren ergibt sich dann folgender Expansionsdrück

$p_E$ p 30 x 1000 = 50 x $p_E$

$p_E$ = 600 mbar abs. = -400 mbar rel.

b) Pumpe am oberen Totpunkt

$V_1$ = 50 cm$^3$

$V_2$ = 0

p = 1000 mbar

Bei der Kompression wird folgender Kompressionsdruck $p_K$ erhalten:

1000 x 50 = $p_K$ x 30

$p_K$ = 1.665 mbar abs., somit 665 mbar rel.

Mit der erfindungsgemäßen Pumpanordnung wird somit ein günstiges Verhältnis von Pump- und Balgvolumen erreicht. Zusätzlich sind die erzeugbaren Drücke durch das Verhältnis der Volumina vorgegeben und somit begrenzt.

Insofern läßt sich die gesamte Pumpanordnung wesentlich kleiner bauen und ist dennoch beim Betrieb völlig zufriedenstellend.

Die erfindungsgemäße Pumpanordnung kann vorteilhafterweise über- und/oder unterdruckkontrolliert werden, wobei die bekannten Ventile hierzu eingesetzt werden. Zusätzlich kann über die Beobachtung des gesamten Druckverhaltens, das vorteilhafterweise in einem Speicher gespeichert wird, die gesamte Anlage überwacht und ggf. abgeschaltet werden.

Weitere Einzelheiten, Vorteile und Merkmale sind in der nachfolgenden Beschreibung unter Bezugnahme auf die Zeichnungen erläutert.

Es zeigen

Fig. 1    eine Pumpanordnung im Querschnitt,

Fig. 2    schematisch eine Infusionsvorrichtung mit der Pumpanordnung,

Fig. 3    schematisch eine weitere Infusionsanordnung mit mehreren Pumpengehäusen und einer Balgpumpe und

Fig. 4    eine spezielle Ausführungsform eines Pumpengehäuses.

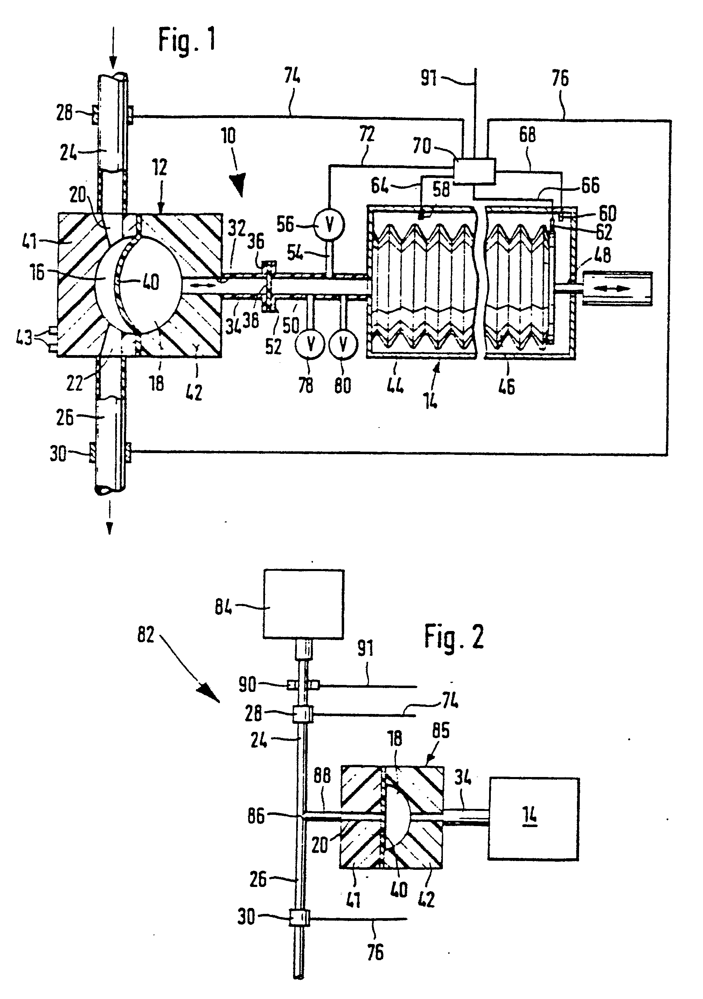

Aus Fig. 1 ist die erfindungsgemäße Pumpanordnung mit 10 ersichtlich. Diese Pumpanordnung 10 besteht im wesentlichen aus einem Pumpengehäuse 12 und einer ein begrenztes Pumpvolumen aufweisenden Pumpe 14, insbesondere einer Kolbenpumpe oder einer Balgpumpe. Letztere ist in Fig. 1 dargestellt.

Das Pumpengehäuse weist zwei Kammern auf, nämlich eine Arbeitskammer 16 für die zu pumpende Flüssigkeit und eine Antriebskammer 18 für das von der Pumpe 16 ein- und auszupumpende Fluid, insbesondere Luft.

Die Arbeitskammer 16 weist wenigstens eine erste Öffnung auf, wobei in Fig. 1 zwei Öffnungen 20 und 22 dargestellt sind, durch die die zu pumpende Flüssigkeit in die erste Pumpkammer eingepumpt und aus dieser herausgepumpt werden kann. Die beiden Öffnungen 20 und 22 sind jeweils mit Schlauchleitungen 24 und 26 verbunden, die mit nicht gezeigten Speichergefäßen bzw. nachgeschalteten Einrichtungen, die mit der zu pumpenden Flüssigkeit beaufschlagt werden, weiter verbunden sind.

Diese Schlauchleitungen 24 und 26 sind mit Absperreinrichtungen 28 und 30, vorzugsweise elektromagnetischen Schlauchklemmen absperrbar, die im Gegentakt betätigt werden. Wenn man davon ausgeht, daß die Leitung 24 als Flüssigkeitszuführleitung und die Leitung 26 als Flüssigkeitsabführleitung dienen soll, so ist die Absperreinrichtung 28 in der Füllphase geöffnet, während die Absperreinrichtung 30 gesperrt ist. In der Entleerungsphase wird dann entsprechend umgeschaltet, also die Klemme 30 geöffnet und die Klemme 28 geschlossen.

Die Antriebskammer 18 ist ebenfalls mit einer zweiten Öffnung 32, die wie die übrigen Öffnungen 20 und 22 das Pumpengehäuse durchsetzt, nach außen hin geöffnet, so daß durch diese Öffnung 32 ein Pumpfluid ein- und ausgepumpt werden kann.

An diese Öffnung 32 schließt sich eine im wesentlichen starre Leitung 34 an, die vorteilhafterweise an ihrem Ende einen Flansch 36 aufweist. Diesen Flansch 36 überspannte vorteilhafterweise eine hydrophobe Membran 38, deren Poren so bemessen sind, daß hierdurch keine Keime in die Leitung 34 und somit in die Antriebskammer 18 eintreten kann. Hierdurch wird sichergestellt, daß bei einem Bruch der nachstehend erläuterten Pumpmembran 40 nicht die üblicherweise steril zu haltende wässrige Flüssigkeit durch die Leitung 34 abfließt und dabei unsteril wird. Somit dient die Membran 38 als Sicherheitseinrichtung für einen Bruch der Pumpenmembran 40.Eine derartige Anordnung ist in der DE-A- 32 05 449 umschrieben.

Diese Pumpenmembran 40 teilt die beiden Pumpkammern 16 und 18 und kann vorteilhafterweise eine bereits vorgeformte gekrümmte Gestalt besitzen. In dieser Ausführungsform kann sie sich ohne Erweiterung jeweils an die Wandungen der Kammern anlegen. In einer weiteren Ausführungsform besitzt die Pumpenmembran 40 eine ebene Ausbildung und kann vorteilhafterweise bereits um einen bestimmten Betrag vorgespannt im Pumpengehäuse 12 angeordnet sein. In diesem Fall erweitert sich die Membran elastisch beim Pumpbetrieb und neigt dazu, bei Entlastung, beispielsweise bei Entlüftung der Pumpe 14 oder Öffnen der Klemmen 28 und 30 in den Ausgangszustand zurückzukehren.

Vorteilhafterweise besteht das Pumpengehäuse 12 aus zwei Gehäusehälften 41 und 42, zwischen die bei der Herstellung die Membran zunächst eingelegt wird und die anschließend miteinander verklebt oder verschweißt werden. Als Material für das Pumpengehäuse wird üblicherweise ein leicht formbares Kunststoffmaterial, beispielsweise PVC, Polyacrylglas oder Polycarbonat gewählt, während als Material für die Pumpenmembran Weich-PVC, Polyethylen oder dgl., sofern die Membran unelastisch ist, oder gummielastische Materialien, wie Silikongummi u.dgl., eingesetzt werden.

Das Pumpvolumen $V_1$ wird durch die Volumina der beiden Kammern 16 und 18 bestimmt. Vorteilhafterweise wird man unterschiedliche Normvolumina einsetzen, je nachdem, welche Pumpraten erzeugt werden sollen.

Weiterhin ist es bevorzugt, daß das Pumpengehäuse 12 einschließlich der mit ihr verbundenen Schläuche 24 und 26 und der Leitung 34 als sog. Wegwerfteil zum Einsatz kommt, das bereits im sterilen Zustand die Produktionsstätte verläßt.

In einer ersten zweckmäßigen Ausführungsform ist die Kontur der Kammern 16 und 18 identisch, wie dies in Fig. 1 gezeigt ist.

Andererseits können jedoch in einer weiteren Ausführungsform die Konturen der Kammern 16 und 18 asymmetrisch sein. In Beispiel 2 ist eine solche asymmetrische Anordnung gezeigt, bei der vorteilhafterweise als Pumpenmembran 40 eine elastische Pumpenmembran eingesetzt wird. In der in Fig. 2 gezeigten Ausführungsform liegt die ebene Membran direkt an der Kammerwand an, so daß praktisch eine zweite Pumpkammer entfällt und die zweite Pumpkammer - wie vorstehend erläutert - wiederum mit der Pumpe 14 in Verbindung ist. Eine derartige Pumpe wird, sofern eine ausreichend elastische Membran gewählt wird, als selbstentleerend bezeichnet.

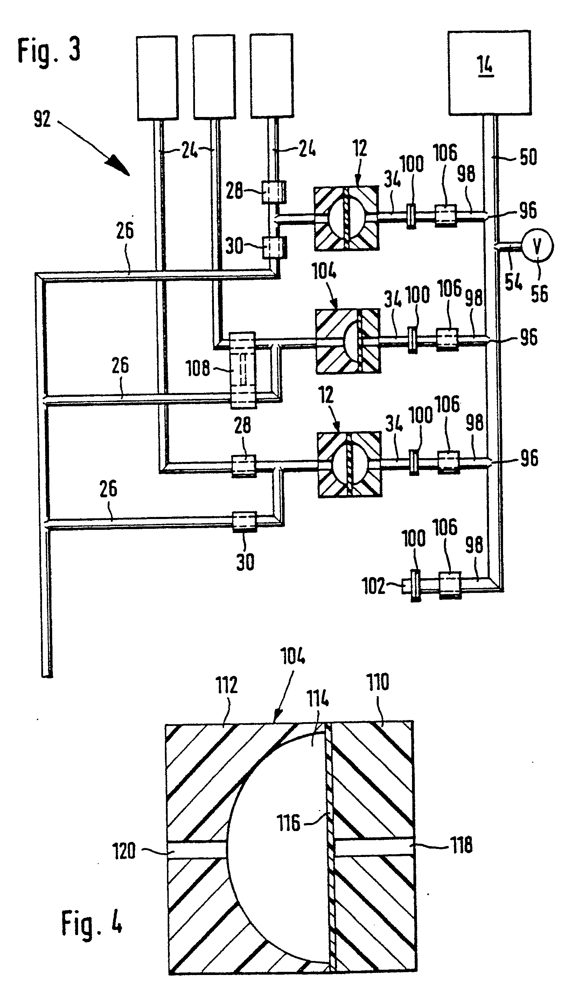

Andererseits kann jedoch auch die Kontur der Kammern 16 und 18 derart ausgebildet sein, daß die Pumpanordnung 10 als selbstansaugend angesehen wird. Eine derartige Ausbildung ist in Fig. 3 in der mittleren Pumpanordnung gezeigt. In diesem Fall liegt die Membran auf der Seite der Pumpe 14 an der ebenen Wand an, so daß die Antriebskammer 18 im nichtbelasteten Zustand entfällt. In einer solchen Ausführungsform wird ebenfalls eine elastische, ggf. vorgespannte Pumpenmembran 40 eingesetzt.

Wie bereits vorstehend festgestellt, kann die Pumpanordnung 10 ein variables Innenvolumen $V_1$ aufweisen. Zur Festlegung der Pumprate wird entweder diese Größe in ein Steuergerät in Verbindung mit dem zu fördernden Volumen eingetippt oder aber das Pumpengehäuse 12 weist in Abhängigkeit von dem Volumen $V_1$ spezielle Kennmarken in Form von Vorsprüngen 43 an der Außenwand auf, die beim Einlegen des Pumpengehäuses 12 in eine nicht gezeigte Steuereinrichtung einen bestimmten Kontakt auslösen, der zur Speicherung des Pumpvolumens $V_1$ im Steuergerät führt. Dies ist insbesondere bei sog. Kassetten der Fall, die in ein derartiges Steuergerät eingelegt werden.

Die Pumpe 14 ist, wie in Fig. 1 gezeigt, als Balgpumpe ausgebildet und besteht demzufolge aus einem Gehäuse 44, in dem ein Balg 46 angeordnet ist, der mittels eines sich hin und her bewegenden Stößels 48 zusammengepreßt oder auseinandergedrückt wird. Das Ende dieses Balges 46 ist über eine starre Leitung 50, deren Ende einen Flansch 52 aufweist, mit dem Pumpengehäuse 12 in Verbindung zu bringen, insbesondere mit der Leitung 34, wobei die beiden Flansche 36 und 52 aneinander befestigt werden.

Vorteilhafterweise ist die Leitung 50 im nicht gekuppelten Zustand verschlossen, so daß hierdurch keine Luft entweichen kann. Dies ist insbesondere dann von Interesse, wenn die Pumpe 14 mit mehreren Pumpengehäusen 12 über ein Leitungssystem verbunden ist, wie dies in Fig. 3 gezeigt ist. In einem solchen Fall ist vorteilhafterweise jeweils die Leitung 50 automatisch geschlossen; wenn keine Verbindung mit einem Pumpengehäuse 12 vorliegt, jedoch geöffnet, was vorteilhafterweise durch eine selbsttätig wirkende Ventilanordnung, wie dies nachstehend erläutert ist, erreicht wird, wenn die Verbindung hergestellt ist.

Von der Leitung 50 geht eine weitere Leitung 54 ab, die mit einem Ventil 56 verschlossen ist. Über dieses Ventil 56 kann die gesamte Pumpanordnung 10 belüftet werden, so daß der jeweils in der Pumpanordnung 10 herrschende Unter- oder Überdruck beseitigt und die gesamte Anordnung auf den Atmosphärendruck eingestellt wird.

Eine besonders bevorzugte Ausführungsform der Erfindung liegt darin, daß die Belüftung der Pumpanordnung 10 jeweils am oberen und unteren Totpunkt der Pumpe 14 vorgenommen wird.

Zu diesem Zweck ist das Gehäuse 44 der Pumpe 14 mit Kontakten 58 und 60 ausgerüstet, die mit einen Kontakt 62 zusammenwirken, der auf dem Balg 46 angeordnet ist. Diese Kontakte sind über Leitungen 64, 66, 68 mit einem Steuergerät 70 verbunden, das wiederum über eine Leitung 72 mit dem Ventil 56 verbunden ist.

Weiterhin ist das Steuergerät über eine Leitung 74 und eine Leitung 76 mit der Absperreinrichtung 28 bzw. 30 verbunden.

Vorteilhafterweise im unteren Totpunkt (komprimierter Balg 46) und im oberen Totpunkt (expandierter Balg) schaltet die Steuereinrichtung 70 jeweils die Klemmen 28 und 30 um, d.h. zum Füllen der Kammer 16 wird die Klemme 28 geöffnet und die Klemme 30 geschlossen, während zum Entleeren der Kammer 16 die Klemme 28 geschlossen und die Klemme 30 geöffnet wird.

Zugleich wird vorteilhafterweise - wie vorstehend erwähnt - jeweils an diesen Totpunkten die Pumpanordnung 10, insbesondere die Antriebskammer 18 und der Innenraum des Balgs 46 durch Öffnen des Ventils 56, was ebenfalls das Steuergerät 70 durch Schließen der Kontakte 56 - 60 veranlaßt, solange geöffnet, bis die Pumpanordnung 10 druckausgeglichen ist. Vorteilhafterweise sind während dieser Öffnungsphase die beiden Klemmen 28 und 30 geschlossen.

Vorteilhafterweise wird die Pumpanordnung mit bestimmten Grenzdrücken sowohl im Unterdruckbereich als auch im Überdruckbereich gefahren. Werden diese Grenzwerte überschritten, dann öffnen die Druckbegrenzungsventile 78 und 80, die einen bestimmten festgelegten Druckgrenzwert während der Pumpphase der Pumpe 14 aufrechterhalten. Diese Druckbegrenzungsventile 78 und 80 werden automatisch geschlossen, wenn die Pumpe ihren oberen oder unteren Totpunkt erreicht hat. Im übrigen ist eine derartige Druckbegrenzungsanordnung in der DE-A- 32 05 449 beschrieben, auf deren Offenbarung ausdrücklich Bezug genommen wird.

In dieser Druckschrift ist ein weiteres Pumpengehäuse beschrieben, bei dem anstelle einer flächigen Pumpenmembran 40 eine schlauchförmige Pumpenmembran eingesetzt wird. Auf die Offenbarung dieser Pumpenmembran sowie der Befestigung dieser Membran in dem Pumpengehäuse wird wiederum Bezug genommen.

Wie bereits eingangs erläutert, hängt bei dieser Anordnung der zu erreichende Unterdruck bzw. Überdruck im wesentlichen vom Verhältnis des Pumpvolumens $V_1$ und des Volumens $V_2$ des ausgezogenen Balges 46 ab. Insofern ist es leicht, den Druckgrenzwert der Druckbegrenzungsventile 78 und 80 festzulegen, der vorteilhafterweise wenigstens 10 % über den jeweiligen Druckgrenzwerten liegt, die aufgrund der gewählten Anordnung festgelegt sind.

Diese Grenzdruckwerte werden so gewählt, daß die eingesetzten Materialien diese Drücke ohne weiteres aushalten können. Sie liegen insofern in einem Bereich von 100 mbar - 3 bar, vorzugsweise 400 mbar - 2 bar.

Andererseits können jedoch aber auch diese Bereiche überschritten werden, sofern entsprechend feste Materialien eingesetzt werden.

Die in Fig. 1 gezeigte Pumpanordnung 10 wird auf folgende Weise betrieben:
Über die Leitung 24 wird von einem nicht gezeigten Behälter das zu fördernde Fluid, beispielsweise Blut oder eine Infusionslösung, dem Pumpengehäuse 12 zugeführt. Hierzu ist die Klemme 28 offen, während die Klemme 30 geschlossen ist. Weiterhin befindet sich die Balgpumpe am unteren Totpunkt, d.h. am linken Anschlag gemäß der in Fig. 1 gezeigten Ausführungsform. Der Druck ist ebenfalls auf Normaldruck gestellt. Anschließend wird der Balg expandiert, d.h. nach rechts bewegt, wobei durch die expandierte Luft die Pumpenmembran 40 ebenfalls nach rechts mitgenommen wird. Hierdurch wird ein Unterdruck im System erzeugt, der zum Einpumpen des Fluids in die Arbeitskammer 16 führt. Die Pumpphase ist dann beendet, wenn das Kammervolumen $V_1$ vollständig mit Fluid gefüllt ist, d.h. wenn sich die Pumpenmembran 40 an die rechte Kammerwand angelegt hat. Beim Anlegen bewegt sich jedoch noch der Balg 46 weiter nach rechts, bis der obere Totpunkt erreicht wird. Sollte hierbei der bestimmte Grenzdruckwert überschritten werden, öffnet automatisch das Druckbegrenzungsventil 78, um einen bestimmten Grenzüberdruckwert konstant zu halten.

Im oberen Totpunkt schaltet dann das Steuergerät 70 zunächst die Klemme 28 zu, öffnet das Ventil 56 zur Einstellung des Drucks auf den Normaldruckwert und öffnet dann die Klemme 30. Zugleich wird die Phasenlage der Pumpe 14 umgeschaltet, die dann in die Kompressionsphase, d.h. in die Abpumpphase überführt wird. Hierdurch wird durch den entstehenden Überdruck die Pumpenmembran solange nach links bewegt, bis sie sich an die Wand der linken Kammerhälfte 16 angelegt hat, d.h. bis der Zustand der völligen Entleerung erreicht ist.

Sollte hier ein bestimmter Überdruck noch während der Pumpphase erreicht werden, öffnet das andere Überdruckbegrenzungsventil 80 und hält diesen Überdruck konstant.

Bei Erreichen des unteren Totpunkts wird dann wiederum das Ventil 56 zur Einstellung auf den Atmosphärendruckwert betätigt.

Anschließend beginnt erneut die Füllphase.

In Fig. 2 ist eine spezielle Infusionsvorrichtung mit 82 gezeigt, wobei für gleiche Teile die gleichen Bezugsziffern wie in Fig. 1 gezeigt werden.

Diese Infusionsvorrichtung 82 weist einen Infusionsbeutel 84 auf, der über die Leitung 24 mit dem Pumpengehäuse 85 verbunden ist, die jedoch nur eine Öffnung 20 aufweist, die zugleich als Zuführ- und Abführöffnung dient. Eine solche Ausführungsform mit nur einer Öffnung gehört zu den bevorzugten Ausführungsformen.

Dementsprechend verzweigt sich also die Leitung 24 bei dem Verzweigungspunkt 86, von dem die Leitung 88, die mit der Öffnung 20 verbunden ist, und die Leitung 26 abgeht.

Gemäß der in Fig. 2 gezeigten Pumpanordnung weist das Pumpengehäuse 85 eine Antriebskammer 18 auf, die der in Fig. 1 gezeigten Antriebskammer 18 entspricht. Ebenfalls entspricht die Pumpenmembran 40 der in Fig. 1 gezeigten Pumpenmembran 40. Es fehlt jedoch vollständig die Arbeitskammer 16 gem. Fig. 1. Demzufolge liegt die ebene Membran 40 völlig an der Wand der Gehäusehälfte 41 an.

Vorteilhafterweise wird in dieser Ausführungsform eine derart elastische Membran gewählt, daß diese durch den hydrostatischen Druck zwangsläufig geöffnet wird, sofern die Flüssigkeitssäule bis in den Beutel 84 reicht. Die elastische Rückstellkraft soll jedoch so bemessen sein, daß sich die Pumpenmembran erst dann erweitert, wenn die Wassersäule wenigstens 10 cm beträgt. Vorteilhafterweise ist dabei die Klemme 28 noch unterhalb dieses Grenzwerts, d.h. unterhalb etwa 10 cm angeordnet, so daß sich keine Luftblasen in dem Pumpsystem bilden können, was gerade bei der Infusion von besonderer Bedeutung ist. Die Infusionsvorrichtung 82 gem. Fig. 2 wird auf folgende Weise betrieben:

Bei geöffneter Klemme 28 drückt die im Beutel 84 befindliche Flüssigkeit die vorteilhafterweise elastische Pumpenmembran 40 solange nach rechts, bis sich die Membran 40 an die Wand der rechten Kammerhälfte 42 angelegt hat.

Während dieser Füllphase kann gemäß einer ersten Ausführungsform die Balgpumpe 14 den Füllvorgang unterstützen, wobei das Ventil 56 gemäß der in Fig. 1 beschriebenen Betriebsweise betätigt wird, d.h. nur an den oberen und unteren Totpunkten geöffnet wird. Gemäß einer zweiten Ausführungsform kann jedoch das Ventil 56 auch während der Füllphase, d.h. Expansion des Balges 46, geöffnet sein, wobei es zu einem selbsttätigen Füllvorgang durch den Druck der Wassersäule kommt. Diese Ausführungsform ist bei der in Fig. 2 gezeigten Infusionsvorrichtung 82 besonders bevorzugt.

Nach erfolgter Füllung wird dann der Balg 46 bei geschlossenem Ventil 56 komprimiert und am unteren Totpunkt belüftet. Hierauf beginnt wiederum die selbsttätige Füllphase.

Es muß nicht besonders hinzugefügt werden, daß anstelle des asymmetrischen Pumpengehäuses gemäß Fig. 2 das symmetrische Pumpengehäuse gemäß Fig. 1 eingesetzt werden kann, sofern die Membran die gleichen elastischen Eigenschaften besitzt. Allerdings kann hier die Rückstellkraft der Membran zu einer Einschleppung von Luftblasen in die Arbeitskammer 16 führen, so daß an der Leitung 28 vorteilhafterweise Luftsensoren 90 angebracht sind, die über die Leitung 91 mit dem Steuergerät 70 verbunden sind und die gesamte Vorrichtung abschalten können.

In Fig. 3 ist eine Pumpvorrichtung 92 gezeigt, die vier Anschlüsse für je vier Pumpengehäuse besitzt, von denen nur drei belegt sind.

Von der Pumpe 14 geht wiederum die Leitung 50 ab, in die über die Leitung 54 das Ventil 56 eingeschaltet ist. Die Leitung 50 weist gemäß der in Fig. 3 gezeigten Ausführungsform vier Verzweigungen 96 auf, von denen Leitungen 98 abgehen, deren Ende jeweils Anschlüsse 100 aufweisen. Diese Anschlüsse 100 sind vorteilhafterweise mit einem Sperrventil 102 ausgerüstet, das beim Konnektieren mit der Leitung 34 geöffnet wird.

Wie in Fig. 3 gezeigt, weist die Pumpvorrichtung 92 zwei Pumpengehäuse 12, wie in Fig. 1 gezeigt, und ein asymmetrisches Pumpengehäuse 104 auf, das nachstehend erläutert wird.

Derartige Pumpvorrichtungen 92 werden besonders vorteilhaft in der Intensivmedizin eingesetzt, bei der der Patient mit mehreren Infusionsleitungen versehen ist.

Sollten jeweils gleiche Infusionsraten erzielt werden, so werden vorteilhafterweise Pumpengehäuse 12, 104 mit gleichem Innenvolumen $V_1$ eingesetzt, wobei die Pumpe entsprechen der gewünschten Rate mit der entsprechenden Geschwindigkeit betrieben wird.

In Abhängigkeit vom Balgvolumen $V_2$ steigt die Pumpengröße $V_1$ vorteilhafterweise entweder um die Faktoren 2, 4, 8 usw. oder um die Faktoren 4, 16, 32 usw. Dabei sollen die Volumina $V_1$ und $V_2$ so ausgelegt sein, daß generell eine kontinuierliche Pumprate ohne größere Pausenzeiten erzielt werden kann.

Wenn jedoch unterschiedliche Pumpraten, d.h. unterschiedliche Infusionsflüssigkeitsmengen den Patienten verabreicht werden sollen, so können teilweise Pumpengehäuse 12, 104 mit unterschiedlichen Volumina eingesetzt werden, teilweise Pumpengehäuse 12 mit gleichen Volumina zur Anwendung kommen, sofern die Pausenzeiten nicht zu groß sind und spezielle Vorkehrungen zur Verminderung der Pumprate getroffen werden. Bei dieser Betriebsweise ist natürlich die Infusionsrate mit der höchsten Pumpenfrequenz geschwindigkeits- und steuerungsbestimmend. Sofern also diese Pumpenfrequenz zu einer überhöhten und somit unerwünschten Pumprate führt, können folgende Vorkehrungen getroffen werden:

Soll beispielsweise die Infusionsflüssigkeit, die in das obere Pumpengehäuse 12 gem. Fig. 3 eingeführt wird, in geringeren Mengen infundiert werden, so ist vorteilhafterweise in der Leitungsverbindung 98 bzw. 34 ein Absperrorgan 106 vorgesehen, das diese Leitungen entsprechend sperrt. So können bestimmte Hübe des Balgs 46 und somit bestimmte Pumpschritte dadurch ausgelassen werden, daß das Absperrorgan 106, vorzugsweise ein Ventil oder eine Schlauchklemme, sofern ein elastischer Schlauch eingesetzt wird, am unteren Totpunkt der Pumpe 14 für einen bestimmten Zeitraum geschlossen wird, beispielsweise solange, bis die Pumpe 14 den oberen Totpunkt erreicht hat.

Anstelle des Absperrorgans 106 kann in einer anderen Ausführungsform die Klemme 30 geschlossen bleiben, wobei bei geöffneter Klemme 28 in den Beutel zurückgepumpt wird oder bei ebenfalls geschlossener Klemme 28 lediglich ein erhöhter Unterdruck in Pumpengehäuse 12 erzeugt wird.

In einer weiteren Ausführungsform ist es auch denkbar, daß beim Expandieren des Balges 46 das Absperrorgan 28 geschlossen bleibt, während das Absperrorgan 30 geöffnet wird. In einem solchen Fall ist jedoch darauf zu achten, daß es zu keinem Rücksaugvorgang von den anderen Abführleitungen der übrigen Pumpengehäuse kommt.

Als Absperrorgane 28 und 30 bzw. 106 kommen die üblichen magnetischen oder mechanischen Klemmen in Frage, wobei kombinierte Klemmen für beide Absperreinrichtungen 28 und 30 bevorzugt sind. Derartige Kombinationsklemmen, bei denen immer nur eine der Klemmen offen ist, während die andere zwangsläufig geschlossen ist, ist mit 108 in Fig. 3 gezeigt. Diese Klemme 108 gem. Fig. 3 wird linear zwischen den beiden Anlagepunkten bewegt. Denkbar ist jedoch auch eine Nocke, die an einer Scheibe angebracht ist, die jeweils um einen bestimmten Betrag von der einen Schließposition in die andere Schließposition überführt wird.

Diese Ventile können also - wie vorstehend erläutert - zwangsgeführt werden oder aber auch gesteuert werden, sofern elektromagnetische Ventile eingesetzt werden.

In Fig. 4 ist vergrößert das asymmetrische Pumpengehäuse 104 gem. Fig 3 gezeigt. Dieses Pumpengehäuse 104 weist eine zweite Kammerhälfte 110 und eine erste Kammerhälfte 112 auf, wobei die Kammerhälfte 110 in Form einer ebenen Platte ausgebildet ist, während die Kammerhälfte 112 eine halbkugelförmige Aushöhlung 114 aufweist. Beide Kammerhälften sind von der Pumpenmembran 116 getrennt. Zusätzlich weisen beide Kammerhälften 110 und 112 Öffnungen 118 und 120 auf.

Gemäß dieser Ausführungsform ist die Öffnung 118 mit der Leitung 34 verbunden, während die Öffnung 120 mit der Leitung 88 verbunden ist.

Wie bereits vorstehend erläutert, ist diese Pumpenform, sofern eine elastisch erweiterbare Membran 116 eingesetzt wird, selbst zurückstellend, wenn sich die Membran 116 völlig an die Innenwand des Pumpengehäuses 112 angelegt hat und die Pumpe 14 komprimiert ist.

In dieser Betriebsweise wird das Ventil 56 vorteilhafterweise am unteren Totpunkt geöffnet, was zur Folge hat, daß sich die Membran 116 in die Ausgangslage zurückbegibt. Hierdurch füllt sich der Raum 114 selbsttätig mit der Pumpflüssigkeit. Somit handelt es sich hier um eine selbst-ansaugende Pumpe.

Falls jedoch die Öffnung 120 mit der Pumpe 14 über die Leitung 34 verbunden ist und die Öffnung 118 mit dem Behälter der zu pumpenden Flüssigkeit über die Leitung 88 verbunden ist, wird die Membran 116 beim Expandieren der Pumpe nach hinten solange mitgenommen, bis sie sich an die Innenwand der Aushöhlung 114 angelegt hat. Im oberen Totpunkt der Pumpe 14 (Balgpumpe oder Kolbenpumpe) erfolgt dann die Belüftung des Systems durch Öffnen des Ventils 56 mit der Folge, daß sich die Membran 116 in ihre Ausgangslage aufgrund ihrer elastischen Rückstellkraft zurückbegibt und die gespeicherte Flüssigkeit durch die Öffnung 118 abdrückt. Somit handelt es sich hier um eine selbst-abfördernde Pumpe.

Die erfindungsgemäßen Pumpanordnungen können vorteilhafterweise mit einer mikroprozessorgesteuerten Auswertungs- und/oder Steuereinheit verbunden sein. Hier können die Verhältnisse der zu fördernden Mengen und die tatsächlichen Mengen programmierbar sein. Weiterhin können diese Einheiten durch die Markierungen 43 bereits auf die speziellen Pumpengehäusetypen programmierbar sein, und zwar unabhängig davon, ob symmetrische oder asymmetrische Pumpengehäuse eingesetzt werden.

## Ansprüche

1. Pumpanordnung für medizinische Zwecke, insbesondere eine Blut- oder Infusionspumpe, mit wenigstens einem Pumpgehäuse (12, 85, 104), das einen Raum aufweist, der von einer undurchlässigen Membran (40, 116) in einer Arbeitskammer (16) und in eine Antriebskammer (18) unterteilt ist, wobei die Arbeitskammer (16) mit wenigstens einer Öffnung (20, 22, 120) zur Zu- und Abführung des zu fördernden Fluids versehen ist und die Antriebskammer (18) mit Luft als Pumpfluid beaufschlagbar und mit einer ein begrenztes Pumpvolumen aufweisenden Pumpe (14) verbunden ist, und mit einem mit der Antriebskammer (18) verbundenen Ventil (56) für den Druckausgleich bei Hubwechsel der Pumpe, dadurch gekennzeichnet, daß ein Steuergerät (70) vorgesehen ist, das vor jedem Hubwechsel der Pumpe (14) das Ventil (56) öffnet und nach Druckausgleich auf Umgebungsdruck schließt und danach den nächsten Hub der Pumpe (14) einleitet.

2. Pumpanordnung nach Anspruch 1, dadurch gekennzeichnet, daß das Ventil (56) mit einem Steuergerät (70) verbunden ist, das durch Schalten der Endkontakte (58, 60) mit einem Schaltkontakt (62) angesteuert wird, der an der Pumpe (14) angeordnet sind.

3. Pumpanordnung nach Anspruch 2, dadurch gekennzeichnet, daß das Steuergerät (70) mit einer stromauf der Arbeitskammer (16) angeordneten Absperreinrichtung (28) und einer stromab der Arbeitskammer (16) angeordneten Absperreinrichtung (30) verbunden ist und diese getaktet öffnet bzw. schließt.

4. Pumpanordnung nach Anspruch 3, dadurch gekennzeichnet, daß die Absperreinrichtung (28) im unteren bzw. oberen Totpunkt der Pumpe (14) geschlossen bzw. geöffnet wird, während die Absperreinrichtung (30) umgekehrt geöffnet bzw. geschlossen wird.

5. Pumpanordnung nach einem der Ansprüche 1 - 4, dadurch gekennzeichnet, daß die Pumpe (14) mit Unterdruck- und Überdruckbegrenzungsventilen (78, 80) ausgerüstet ist.

6. Pumpanordnung nach einem der Ansprüche 1 - 5, dadurch gekennzeichnet, daß die Verbindungsleitung zwischen der Antriebskammer (18) des Pumpengehäuses (12) mit der Pumpe (14) durch ein Absperrorgan (106) gesteuert geöffnet bzw. geschlossen wird.

7. Pumpanordnung nach einem der Ansprüche 1 - 6, dadurch gekennzeichnet, daß das Pumpengehäuse (12, 85, 104) aus einer ersten und einer zweiten Gehäusehälfte (41 bzw. 42; 112 bzw. 110) besteht.

8. Pumpanordnung nach Anspruch 7, dadurch gekennzeichnet, daß die erste Gehäusehälfte (41; 112) wenigstens eine erste Öffnung (20, 22, 120) für die Zu- und Abführung der zu pumpenden Flüssigkeit aufweist und die zweite Gehäusehälfte (42) eine andere Öffnung (32, 118) für das Pumpfluid aufweist.

9. Pumpanordnung nach Anspruch 8, dadurch gekennzeichnet, daß die andere Öffnung (32, 118) mit einer im wesentlichen starren Leitung (34) verbunden ist, deren Ende mit einer hydrophoben, Keime nicht durchlassenden Membran (38) verschlossen ist.

10. Pumpanordnung nach einem der Ansprüche 1 - 9, dadurch gekennzeichnet, daß das Pumpengehäuse (12, 104) ein Kunststoffwegwerfteil ist und volumenabhängige Kennmarken aufweist.

11. Pumpanordnung nach einem der Ansprüche 1 - 10, dadurch gekennzeichnet, daß die Pumpe (14) über eine Leitung (50) mit dem Pumpengehäuse (12, 104) verbindbar ist, die ein Absperrventil (102) aufweist, das im ungekuppelten Zustand die Leitung (50) verschließt.

## Claims

1. Pump arrangement for medical purposes, in particular blood or infusion pump, comprising at least one pump housing (12, 85, 104) having a room which is divided by an impermeable diaphragm (40, 116) into a work chamber (16) and a drive chamber (18) with the work chamber (16) being provided with at least one opening (20, 22, 120) for the supply and discharge of the fluid to be conveyed and said drive chamber (18) being adapted to be subjected to air as pumping fluid and coupled with a pump (14) having a limited pumping volume, and comprising a valve (56) connected to said drive chamber (18) for pressure compensation upon stroke change of the pump, characterized in that a control means (70) is provided which opens the valve (56) before each stroke change of said pump (14) and which closes said valve after pressure equalization with ambient pressure and which thereupon initiates the subsequent stroke of said pump (14).

2. Pump arrangement according to claim 1, characterized in that said valve (56) is connected to a control means (70) which is controlled through switching the end contacts (58, 60) with a switching contact (62) being arranged at the pump (14).

3. Pump arrangement according to claim 2, characterized in that said control means (70) is connected to a first shut-off means (28) disposed upstream of the work chamber (16) and a second shut-off means (30) disposed downstream of the work chamber (16) and opens and closes said shut-off means in a chocked manner.

4. Pump arrangement according to claim 3, characterized in that the shut-off means (28) is closed or opened at the bottom or top deadcenter of said pump (14) whereas the shut-off means (30) is conversely opened or closed.

5. Pump arrangement according to any of claims 1-4, characterized in that said pump (14) is equipped with reduced pressure and excess pressure limiting valves (78, 80).

6. Pump arrangement according to any of claims 1-5, characterized in that the connecting conduit between said drive chamber (18) of said pump housing (12) and the pump (14) is opened or closed controlled by a shut-off means (106).

7. Pump arrangement according to any of claims 1-6, characterized in that said pump housing (12, 85, 104) comprises a first and a second housing half (41 resp. 42; 112 resp. 110).

8. Pump arrangement according to claim 7, characterized in that said first housing half (41; 112) comprises at least one first opening (20, 22, 120) for the supply and discharge of the liquid to be pumped and that said second housing half (42) comprises another opening (32, 118) for the pumping fluid.

9. Pump arrangement according to claim 8, characterized in that said other opening (32, 118) is connected to a substantially rigid conduit (34) the end of which is sealed with a hydrophobic membrane (38) impermeable to germs.

10. Pump arrangement according to any of claims 1-9, characterized in that said pump housing (12, 104) is a plastic disposable component and has code markings depending on the volume.

11. Pump arrangement according to any of claims 1-10, characterized in that said pump (14) is connectable to said pump housing (12, 104) via said conduit (50) having a shut-off valve (102) for closing said conduit (50) in disconnected condition.

## Revendications

1. Ensemble de pompe à usage médical, notamment pompe à sang ou pompe d'injection, comprenant au moins un carter de pompe (12,85,104), qui possède un espace subdivisé par une membrane imperméable (40,116) en une chambre de travail (16) et en une chambre d'entraînement (18), et dans lequel la chambre de travail (16) comporte au moins une ouverture (20,22,120) utilisée pour l'amenée et l'évacuation du fluide à entraîner, et la chambre d'entraînement (18) peut être chargée par de l'air en tant que fluide d'entraînement et est raccordée à une pompe (14) possédant une cylindrée limitée, le dispositif comportant une soupape (56) reliée à la chambre d'entraînement (18) pour réaliser la compensation de pression lors de l'inversion de la course de la pompe, caractérisé en ce qu'il est prévu un appareil de commande (70) qui ouvre la soupape (56) avant chaque inversion de la course de la pompe (14) et ferme cette soupape après la compensation de pression réalisant un équilibrage à la pression ambiante, puis déclenche la course immédiatement suivante de la pompe (14).

2. Dispositif de pompe selon la revendication 1, caractérisé en ce que la soupape (56) est raccordée à un appareil de commande (70), qui est commandé au moyen d'une commutation des contacts d'extrémité (58,60) avec un contact de commutation (62) qui est installé sur la pompe (14).

3. Dispositif de pompe selon la revendication 2, caractérisé en ce que l'appareil de commande (70) est raccordé à un dispositif de blocage (28) disposé en amont de la chambre de travail (16) et un dispositif de blocage (30) disposé en aval de la chambre de travail (16) et ouvre ou ferme ces dispositifs de blocage de façon cadencée.

4. Dispositif de pompe selon la revendication 3, caractérisé en ce que le dispositif de blocage (28) se ferme ou s'ouvre au niveau du point mort bas ou au niveau du point mort haut de la pompe (14), tandis que le dispositif de blocage (30) se ferme ou s'ouvre de façon inverse.

5. Dispositif de pompe selon l'une des revendications 1-4, caractérisé en ce que la pompe (14) est équipée de soupapes (78,80) de limitation de la dépression et de limitation de la surpression.

6. Dispositif de pompe selon l'une des revendications 1-5, caractérisé en ce que la canalisation de liaison entre la chambre d'entraînement (18) du carter de pompe (12) et la pompe (14) est ouverte ou fermée d'une manière commandée par un organe de blocage (106).

7. Dispositif de pompe selon l'une des revendications 1-6, caractérisé en ce que le carter de pompe (12,85,104) est constitué par des première et seconde moitiés (41 ou 42; 112 ou 110).

8. Dispositif de pompe selon la revendication 7, caractérisé en ce que la première moitié (41;112) du carter possède au moins une ouverture (20,22,120) pour l'amenée et l'évacuation du liquide devant être pompé et que la seconde moitié (42) du carter possède une autre ouverture (32,118) pour le fluide d'entraînement.

9. Dispositif de pompe selon la revendication 8, caractérisé en ce que l'autre ouverture (32,118) est raccordée à une canalisation sensiblement rigide (34), dont l'extrémité est fermée par une membrane hydrophobe (38) imperméable aux germes.

10. Dispositif de pompe selon l'une des revendications 1-9, caractérisé en ce que le carter de pompe (12, 104) est une pièce en matière plastique jetable et possède des marques caractéristiques, qui dépendent du volume.

11. Dispositif de pompe selon l'une des revendications 1-10, caractérisé en ce que la pompe (14) peut être raccordée par une canalisation (50) au carter de pompe (12, 104), qui possède une soupape de blocage (102), qui, à l'état désaccouplé, ferme la canalisation (50).

EP 0 156 211 B1

Fig. 1

Fig. 2

11

Fig. 3

Fig. 4